# EUROPEAN PATENT APPLICATION

(11) **EP 4 730 341 A1**
(43) Date of publication of application: **22.04.2026**
(21) Application number: 24845489.4
(22) Date of filing: 17.07.2024
(51) Int. Cl.: G16B 40/00, G16B 30/00

(54) **MEDICINE DEVELOPMENT SUPPORT DEVICE, OPERATION METHOD FOR MEDICINE DEVELOPMENT SUPPORT DEVICE, OPERATION PROGRAM FOR MEDICINE DEVELOPMENT SUPPORT DEVICE, LEARNING DEVICE, OPERATION METHOD FOR LEARNING DEVICE, AND OPERATION PROGRAM FOR LEARNING DEVICE**

(30) Priority: 26.07.2023 JP 2023122002
(71) Applicant: FUJIFILM Corporation, Tokyo 106-8620 (JP)
(72) Inventor: NISHINO, Toru, Ashigarakami-gun, Kanagawa 258-8577 (JP)
(74) Representative: Hoffmann Eitle
(86) International application number: PCT/JP2024/025597
(87) International publication number: WO 2025/023110

(57) **Abstract**

A medicament development support device including: a processor, in which the processor acquires sequence information of amino acid residues constituting a substance derived from amino acids included in a medicament, and physical property information of the amino acid residues, uses a substance language model that has been subjected to pre-training and fine-tuning by using learning data including learning sequence information and learning physical property information, and inputs the sequence information and the physical property information to the substance language model to cause the substance language model to output a prediction result related to the substance, and presents the prediction result to a user.

## Description

### BACKGROUND OF THE INVENTION

### 1. Field of the Invention

The present disclosed technology relates to a medicament development support device, an operation method of a medicament development support device, an operation program of a medicament development support device, a learning device, an operation method of a learning device, and an operation program of a learning device.

### 2. Description of the Related Art

Recently, medicaments such as biopharmaceuticals, peptide medicaments, and nucleic acid medicaments have attracted attention due to high drug efficacy and low side effects. For example, a biopharmaceutical has a protein such as interferon or an antibody as an active ingredient. The biopharmaceutical is preserved in a preservation solution. It is important to prescribe a preservation solution (also referred to as formulation formula) suitable for the biopharmaceutical in order to stably maintain the quality of the biopharmaceutical.

In the field of natural language processing (NLP), a language model such as bidirectional encoder representations from transformers (BERT) using a transformer encoder has attracted attention. In the language model, masked language modeling (MLM) and next sentence prediction (NSP) are performed as pre-training. MLM is a so-called cloze test for predicting which term phrase fits in a masked part of a training input text in which a part of term phrases is masked. The NSP is processing of determining whether or not two sentences are semantically consecutive sentences. After such pre-training, fine-tuning (hereinafter, abbreviated as fine-tuning (FT)) corresponding to a desired natural language processing task is performed. The term phrase is a single term (word) and/or a phrase consisting of a combination of one or more single terms.

Various techniques have been proposed in which sequence information of amino acid residues constituting a protein is treated as a sentence to contribute to the development of biopharmaceuticals by using a language model. For example, in Danqing Wang, et al. "On Pre-trained Language Models for Antibody" Published as a conference paper at ICLR 2023 January 31, 2023. (hereinafter, referred to as Non-Patent Document 1), a technique of performing pre-training of a language model by using a prediction task of a germline cell lineage of a protein (antibody) and a prediction task of a mutation site of the protein is described. In addition, in JP2023-022060A (hereinafter, referred to as Patent Document 1), a technique of performing pre-training of a language model based on sequence information of amino acid residues constituting a protein, functional information of the protein, and structural information of the protein is described. Examples of the structural information include information based on an extraction result of a point group consisting of heavy atoms of the protein. The functional information is described only as "text description information of a function of a protein", and specific examples thereof are not described. The language model to which the sequence information of amino acid residues constituting the protein is applied is called a protein language model.

### SUMMARY OF THE INVENTION

The amino acid residues constituting a substance derived from amino acids, such as a protein, have physical properties such as hydrophilicity/hydrophobicity, surface exposure area, and charge state that differ for each type. In addition, such physical properties affect, for example, an appropriate formulation formula for stabilizing the quality of a biopharmaceutical. However, in Non-Patent Document 1 and Patent Document 1, the physical properties of the amino acid residues are not considered. Therefore, there is a possibility that the prediction accuracy of the protein language model is not sufficient.

One embodiment according to the disclosed technology provides a medicament development support device, an operation method of a medicament development support device, an operation program of a medicament development support device, a learning device, an operation method of a learning device, and an operation program of a learning device, in which it is possible to improve prediction accuracy of a substance language model to which sequence information of amino acid residues constituting a substance derived from amino acids, such as a protein, is applied.

The medicament development support device according to the disclosed technology includes a processor, in which the processor acquires sequence information of amino acid residues constituting a substance derived from amino acids included in a medicament, and physical property information of the amino acid residues, uses a substance language model that has been subjected to pre-training and fine-tuning by using learning data including learning sequence information and learning physical property information, and inputs the sequence information and the physical property information to the substance language model to cause the substance language model to output a prediction result related to the substance, and presents the prediction result to a user.

It is preferable that the substance language model is a model that has been subjected to the pre-training by using the learning data in which at least one of a portion of the sequence information or a portion of the physical property information is masked.

It is preferable that the physical property information is at least one of a numerical value representing hydrophilicity/hydrophobicity of the amino acid residue, a surface exposure area of the amino acid residue, or data representing a charge state of the amino acid residue.

It is preferable that the prediction result includes information related to a formulation of a preservation solution of the medicament.

It is preferable that the substance is any of a protein, a peptide, or a nucleic acid.

It is preferable that the protein is an antibody.

An operation method of a medicament development support device according to the disclosed technology includes acquiring sequence information of amino acid residues constituting a substance derived from amino acids included in a medicament, and physical property information of the amino acid residues, using a substance language model that has been subjected to pre-training and fine-tuning by using learning data including learning sequence information and learning physical property information, and inputting the sequence information and the physical property information to the substance language model to cause the substance language model to output a prediction result related to the substance, and presenting the prediction result to a user.

An operation program of a medicament development support device according to the disclosed technology causes a computer to execute a process including: acquiring sequence information of amino acid residues constituting a substance derived from amino acids included in a medicament, and physical property information of the amino acid residues; using a substance language model that has been subjected to pre-training and fine-tuning by using learning data including learning sequence information and learning physical property information; and inputting the sequence information and the physical property information to the substance language model to cause the substance language model to output a prediction result related to the substance; and presenting the prediction result to a user.

A learning device according to the disclosed technology includes a processor, in which the processor acquires learning data including sequence information of amino acid residues constituting a substance derived from amino acids included in a medicament, and physical property information of the amino acid residues, and performs pre-training and fine-tuning of a substance language model that outputs a prediction result related to the substance by using the learning data.

It is preferable that the learning data used for the pre-training is data in which at least one of a portion of the sequence information or a portion of the physical property information is masked.

An operation method of a learning device according to the disclosed technology includes acquiring learning data including sequence information of amino acid residues constituting a substance derived from amino acids included in a medicament, and physical property information of the amino acid residues, and performing pre-training and fine-tuning of a substance language model that outputs a prediction result related to the substance by using the learning data.

An operation program of a learning device according to the disclosed technology causes a computer to execute a process including: acquiring learning data including sequence information of amino acid residues constituting a substance derived from amino acids included in a medicament, and physical property information of the amino acid residues; and performing pre-training and fine-tuning of a substance language model that outputs a prediction result related to the substance by using the learning data.

According to the disclosed technology, it is possible to provide a medicament development support device, an operation method of a medicament development support device, an operation program of a medicament development support device, a learning device, an operation method of a learning device, and an operation program of a learning device, in which it is possible to improve prediction accuracy of a substance language model to which sequence information of amino acid residues constituting a substance derived from amino acids, such as a protein, is applied.

### BRIEF DESCRIPTION OF THE DRAWINGS

FIG. 1 is a diagram showing a medicament development support system.
FIG. 2 is a diagram showing sequence information.
FIG. 3 is a diagram showing physical property information.
FIG. 4 is a block diagram showing a computer constituting a learning device, a medicament development support device, and a user terminal.
FIG. 5 is a block diagram showing a processing unit of a CPU of the learning device.
FIG. 6 is a diagram showing a past data group.
FIG. 7 is a diagram showing a process of a generation unit.
FIG. 8 is a diagram showing a masking process on the sequence information.
FIG. 9 is a diagram showing a masking process on the physical property information.
FIG. 10 is a diagram showing a first_1 learning data group.
FIG. 11 is a diagram showing a first_2 learning data group.
FIG. 12 is a diagram showing a second learning data group.
FIG. 13 is a diagram showing a process of a pre-training unit.
FIG. 14 is a diagram showing a process in the MLM.
FIG. 15 is a diagram showing a process in the NSP.
FIG. 16 is a diagram showing a process in the FT.
FIG. 17 is a block diagram showing a processing unit of a CPU of the medicament development support device.
FIG. 18 is a diagram showing a process of a prediction unit.
FIG. 19 is a block diagram showing a processing unit of a CPU of the user terminal.
FIG. 20 is a diagram showing an information input screen.
FIG. 21 is a diagram showing a prediction result display screen.
FIG. 22 is a flowchart showing a processing procedure of the learning device.
FIG. 23 is a flowchart showing a processing procedure of the medicament development support device.

### DESCRIPTION OF THE PREFERRED EMBODIMENTS

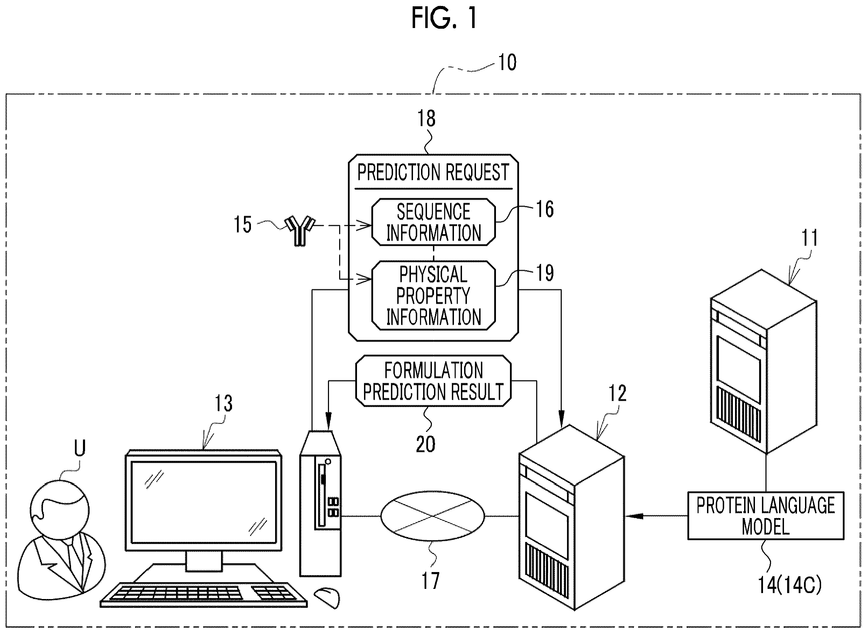

As shown in FIG. 1 as an example, a medicament development support system 10 is a system that supports the development of a biopharmaceutical, and includes a learning device 11, a medicament development support device 12, and a user terminal 13. The learning device 11 generates a protein language model 14 and transmits the protein language model 14 to the medicament development support device 12. The protein language model 14 is a language model based on BERT. The protein language model 14 predicts information that contributes to the development of the biopharmaceutical by treating sequence information 16 of the amino acid residues constituting a protein that is an active ingredient of the biopharmaceutical as a sentence. Here, the protein is an antibody 15. In addition, the information that contributes to the development of the biopharmaceutical is information related to an appropriate formulation formula of a preservation solution for stabilizing the quality of the biopharmaceutical. The biopharmaceutical is an example of a "medicament" according to the disclosed technology. In addition, the antibody 15 is an example of a "substance derived from amino acids" and a "protein" according to the disclosed technology, and the protein language model 14 is an example of a "substance language model" according to the disclosed technology.

The medicament development support device 12 and the user terminal 13 are connected to each other via a network 17. The user terminal 13 is installed in a pharmaceutical company that develops the biopharmaceutical or an institution that receives a development business of the biopharmaceutical from the pharmaceutical company, that is, a contract research organization (CRO). The user terminal 13 is operated by a user U who is involved in the development of the biopharmaceutical in the pharmaceutical company or the CRO. The network 17 is, for example, a wide area network (WAN) such as the Internet or a public communication network. In FIG. 1, only one user terminal 13 is connected to the medicament development support device 12, but in practice, a plurality of user terminals 13 of a plurality of pharmaceutical companies or CROs are connected to the medicament development support device 12.

The user terminal 13 transmits a prediction request 18 to the medicament development support device 12. The prediction request 18 is a request for the medicament development support device 12 to predict information related to an appropriate formulation formula of a preservation solution of the biopharmaceutical. The prediction request 18 includes the sequence information 16 of the amino acid residues constituting the antibody 15 and physical property information 19 of the amino acid residues. The sequence information 16 of the amino acid residues is identified by an experiment. The physical property information 19 is identified by an experiment or a simulation. Although not shown, the prediction request 18 also includes a terminal identification data (ID) or the like for uniquely identifying the user terminal 13 which is a transmission source of the prediction request 18.

In a case in which the prediction request 18 is received, the medicament development support device 12 predicts the information related to the appropriate formulation formula of the preservation solution of the biopharmaceutical by using the protein language model 14. Then, a formulation prediction result 20 that is a result thereof is delivered to the user terminal 13 that is the transmission source of the prediction request 18. In a case in which the formulation prediction result 20 is received, the user terminal 13 shows the formulation prediction result 20 to the user U. The formulation prediction result 20 is an example of a "prediction result" according to the present disclosed technology.

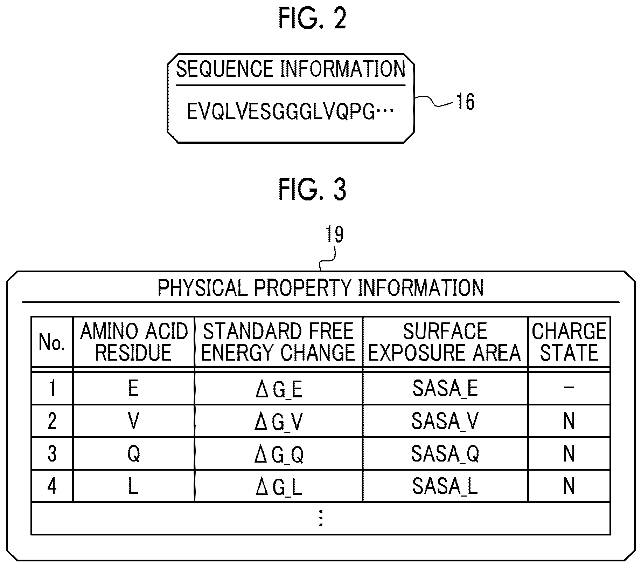

As shown in FIG. 2 as an example, the sequence information 16 of the amino acid residues is described from an amino terminal toward a carboxyl terminal using an abbreviation of one character of an alphabet representing the amino acid residues in an order of peptide bonds of the amino acid residues constituting the antibody 15. Since there are about 450 amino acid residues constituting the antibody 15, the alphabet of the sequence information 16 of the amino acid residues is also about 450. For example, the abbreviation "E" is glutamic acid, "L" is leucine, and "G" is glycine. Such an amino acid residue sequence is also called a primary structure.

As shown in FIG. 3 as an example, the physical property information 19 is information in which a standard free energy change ΔG, a solvent accessible surface area (SASA), and a charge state for each amino acid residue are registered in an order of the amino acid residue sequence. In a case in which the standard free energy change ΔG is positive, it represents that the amino acid residue is hydrophilic, and in a case in which the standard free energy change ΔG is negative, it represents that the amino acid residue is hydrophobic. That is, the standard free energy change ΔG is an example of a "numerical value representing hydrophilicity/hydrophobicity of an amino acid residue" according to the disclosed technology. In a case in which the value of the solvent accessible surface area SASA is larger, it represents that the stability of the amino acid residue is lower. Any of "+(positive)", "-(negative)", and "N (neutral)" is registered as the charge state. These "+(positive)", "-(negative)", and "N (neutral)" are examples of "data representing a charge state of amino acid residues" according to the disclosed technology.

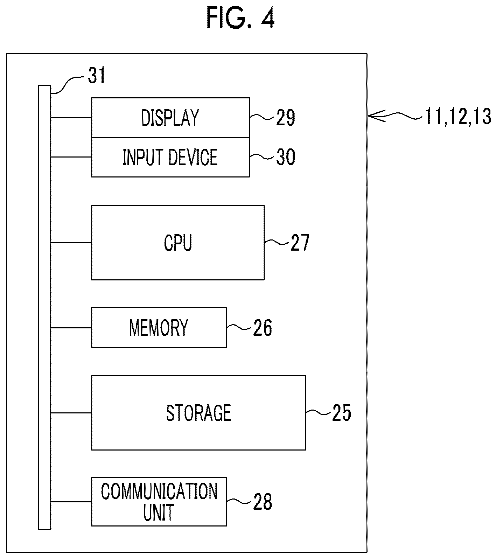

As shown in FIG. 4 as an example, the computer constituting the learning device 11, the medicament development support device 12, and the user terminal 13 basically has the same configuration, and includes a storage 25, a memory 26, a central processing unit (CPU) 27, a communication unit 28, a display 29, and an input device 30. These units are connected to each other through a busline 31.

The storage 25 is a hard disk drive that is built in the computer constituting the learning device 11, the medicament development support device 12, and the user terminal 13 or is connected to the computer through a cable or a network. Alternatively, the storage 25 is a disk array obtained by connecting a plurality of hard disk drives. The storage 25 stores a control program such as an operating system, various application programs (hereinafter, referred to as an application program (AP)), various types of data associated with these programs, and the like. A solid state drive may be used instead of the hard disk drive.

The memory 26 is a work memory for executing processing via the CPU 27. The CPU 27 loads the programs stored in the storage 25 into the memory 26 and executes processing in accordance with the programs. Accordingly, the CPU 27 controls each unit of the computer in an integrated manner. The CPU 27 is an example of a "processor" according to the disclosed technology. The memory 26 may be incorporated in the CPU 27.

The communication unit 28 is a network interface that performs control of transmitting various types of information via a network 17 and the like. The display 29 displays various screens. The various screens have an operation function by a graphical user interface (GUI). The computer constituting the learning device 11, the medicament development support device 12, and the user terminal 13 receives an input of an operation instruction from the input device 30 through various screens. The input device 30 is a keyboard, a mouse, a touch panel, a microphone for audio input, or the like.

In the following description, each unit (the storage 25 and the CPU 27) of the computer constituting the learning device 11 is distinguished by adding a subscript "A" to a reference numeral, each unit (the storage 25 and the CPU 27) of the computer constituting the medicament development support device 12 is distinguished by adding a subscript "B" to a reference numeral, and each unit (the storage 25, the CPU 27, the display 29, and the input device 30) of the computer constituting the user terminal 13 is distinguished by adding a subscript "C" to a reference numeral.

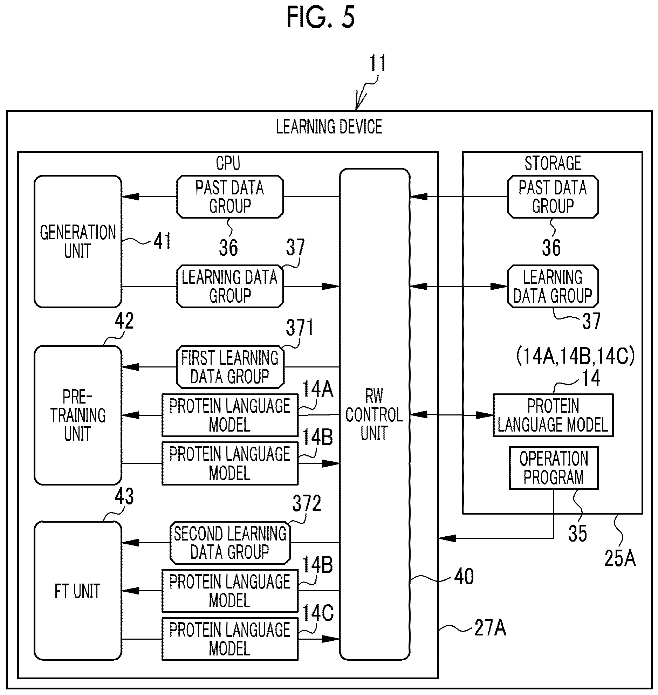

As illustrated in FIG. 5 as an example, the storage 25A of the learning device 11 stores an operation program 35. The operation program 35 is an AP for causing the computer to function as the learning device 11. That is, the operation program 35 is an example of "an operation program of a learning device" according to the technology of the present disclosure. The storage 25A also stores the protein language model 14, a past data group 36, a learning data group 37, and the like.

In a case in which the operation program 35 is activated, the CPU 27A of the computer constituting the learning device 11 functions as an RW control unit 40, a generation unit 41, a pre-training unit 42, and an FT unit 43 in cooperation with the memory 26 and the like.

The RW control unit 40 controls storage of various types of data in the storage 25A and readout of various types of data from the storage 25A. For example, the RW control unit 40 reads out the past data group 36 from the storage 25A and outputs the read past data group 36 to the generation unit 41. In addition, the RW control unit 40 reads out a first learning data group 371 of the learning data group 37 from the storage 25A and outputs the read first learning data group 371 to the pre-training unit 42. Similarly, the RW control unit 40 reads out a second learning data group 372 of the learning data group 37 from the storage 25A and outputs the read second learning data group 372 to the FT unit 43. Further, the RW control unit 40 reads out the protein language model 14 from the storage 25A and outputs the protein language model 14 to the pre-training unit 42 or the FT unit 43.

The generation unit 41 generates the learning data group 37, which is a set of learning data for training the protein language model 14, based on the past data group 36. The generation unit 41 outputs the learning data group 37 to the RW control unit 40. The RW control unit 40 stores the learning data group 37 in the storage 25A.

The pre-training unit 42 performs pre-training of the protein language model 14 by using the first learning data group 371. The pre-training unit 42 outputs the protein language model 14 after the pre-training to the RW control unit 40. The RW control unit 40 stores the protein language model 14 after the pre-training in the storage 25A. In the following description, the protein language model 14 before the pre-training is referred to as a protein language model 14A, and the protein language model 14 after the pre-training is referred to as a protein language model 14B.

The FT unit 43 performs the FT of the protein language model 14B by using the second learning data group 372. The FT unit 43 outputs the protein language model 14B after the FT to the RW control unit 40. The RW control unit 40 stores the protein language model 14B after the FT in the storage 25A. In the following description, the protein language model 14B after the FT is referred to as a protein language model 14C. The protein language model 14C is transmitted from the learning device 11 to the medicament development support device 12. In addition, the CPU 27A is also constructed with an instruction reception unit that receives various operation instructions from the input device 30, in addition to each of the processing units 40 to 43.

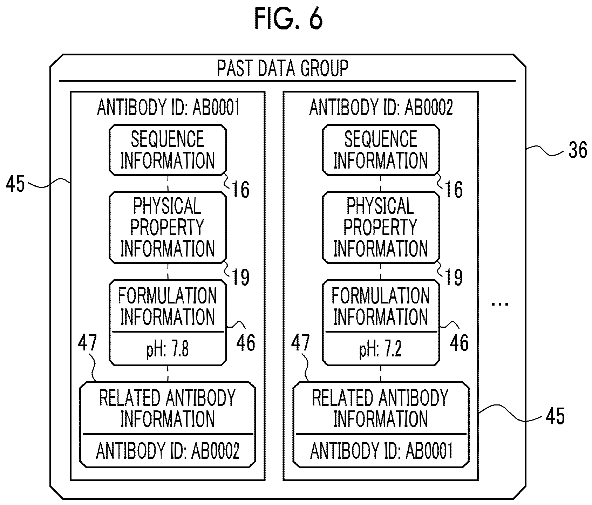

As shown in FIG. 6 as an example, the past data group 36 is a set of past data 45 of a plurality of biopharmaceuticals developed in the past. The past data 45 is individually identified by an antibody identification data (ID) of the antibody 15 included in the biopharmaceutical. The past data 45 may be acquired from a public database of the biopharmaceutical or may be acquired from the biopharmaceutical developed in the past in the pharmaceutical company or the CRO. In addition, the past data 45 may be composed of both the public information and information accumulated independently in the pharmaceutical company or the CRO.

The past data 45 includes formulation information 46 and related antibody information 47 in addition to the sequence information 16 and the physical property information 19. The formulation information 46 is information on a formulation formula adopted in the preservation solution of the biopharmaceutical, and here, a hydrogen ion exponent (pH

(Potential of Hydrogen)) value of the preservation solution is exemplified. The related antibody information 47 registers an antibody ID of the antibody 15 related to the antibody 15 included in the biopharmaceutical. The related antibody 15 is an antibody 15 having a similar structure, an antibody 15 having the same target organ, disease, or the like, and the like.

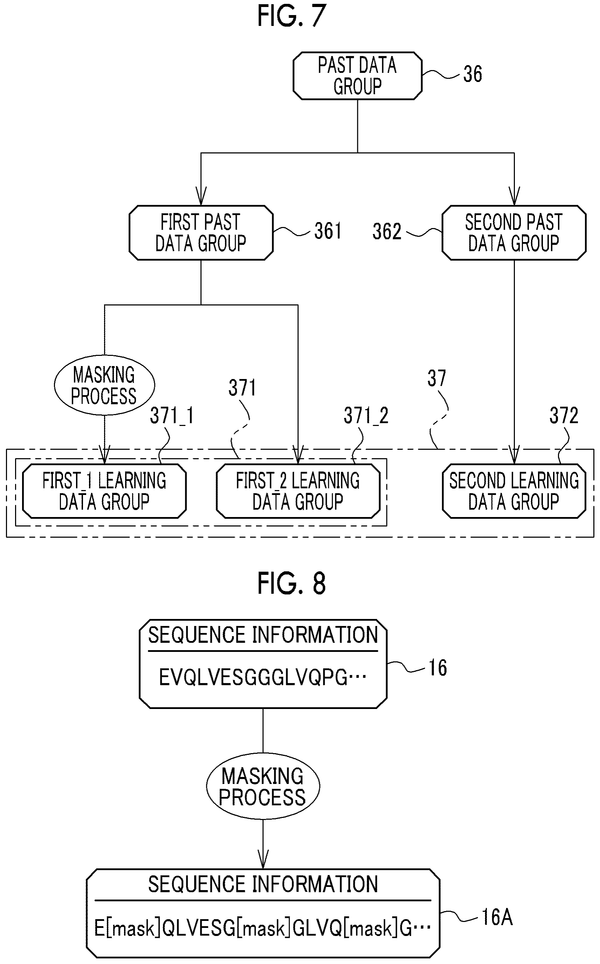

As shown in FIG. 7 as an example, the generation unit 41 first randomly distributes the plurality of pieces of past data 45 of the past data group 36 into a first past data group 361 and a second past data group 362. In this case, the generation unit 41 sets the number of pieces of past data 45 in the first past data group 361 to be larger than the number of pieces of past data 45 in the second past data group 362. For example, 80% of the plurality of pieces of past data 45 of the past data group 36 is distributed to the first past data group 361, and the remaining 20% is distributed to the second past data group 362. The past data 45 of the first past data group 361 and the past data 45 of the second past data group 362 may partially overlap with each other.

The generation unit 41 generates the first learning data group 371 from the first past data group 361. In addition, the generation unit 41 generates the second learning data group 372 from the second past data group 362. The first learning data group 371 and the second learning data group 372 constitute the learning data group 37.

The first learning data group 371 is composed of a first_1 learning data group 371_1 and a first_2 learning data group 371_2. The generation unit 41 generates the first_1 learning data group 371_1 by performing the masking process on the past data 45 of the first past data group 361.

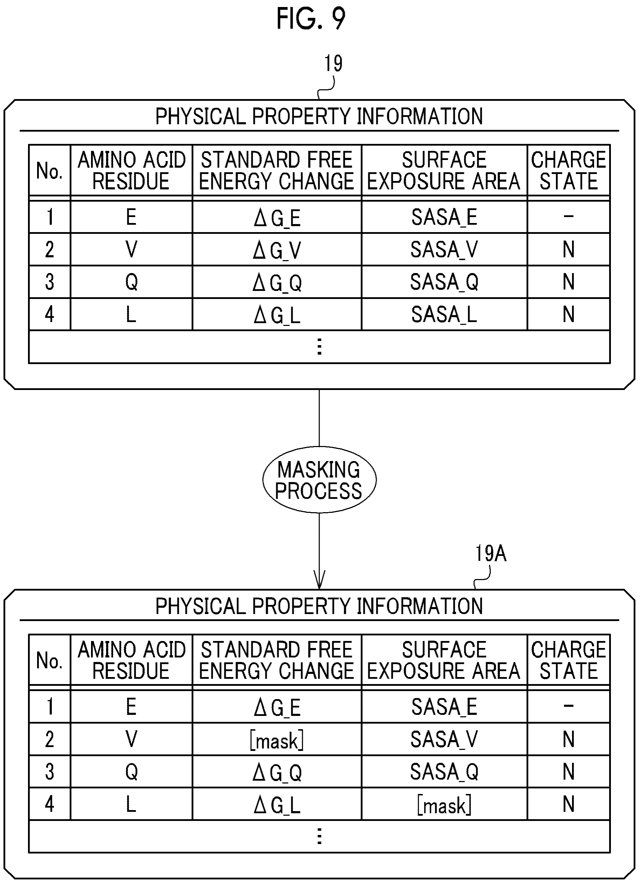

As shown in FIGS. 8 and 9 as an example, the masking process is a process of regarding each of the alphabet of the sequence information 16, the standard free energy change ΔG, the solvent accessible surface area SASA, and the charge state of the physical property information 19 as one token, and masking the alphabet of the sequence information 16 (FIG. 8) or masking the standard free energy change ΔG, the solvent accessible surface area SASA, and the charge state of the physical property information 19 (FIG. 9).

The masking process is performed according to a preset masking condition. The masking condition is, for example, to randomly mask 15% of the alphabet of the sequence information 16, the standard free energy change ΔG, the solvent accessible surface area SASA, and the charge state of the physical property information 19. A case is considered in which the total number of the alphabet of the sequence information 16, the standard free energy change ΔG, the solvent accessible surface area SASA, and the charge state of the physical property information 19 is, for example, 2000. In this case, since 2000 × 0.15 = 300, the masking condition is satisfied by masking at least 300. Since the masking is random, the masking process may not be performed on the sequence information 16, or the masking process may not be performed on the physical property information 19. In the following description, the sequence information 16 after the masking process is referred to as sequence information 16A, and the physical property information 19 after the masking process is referred to as physical property information 19A.

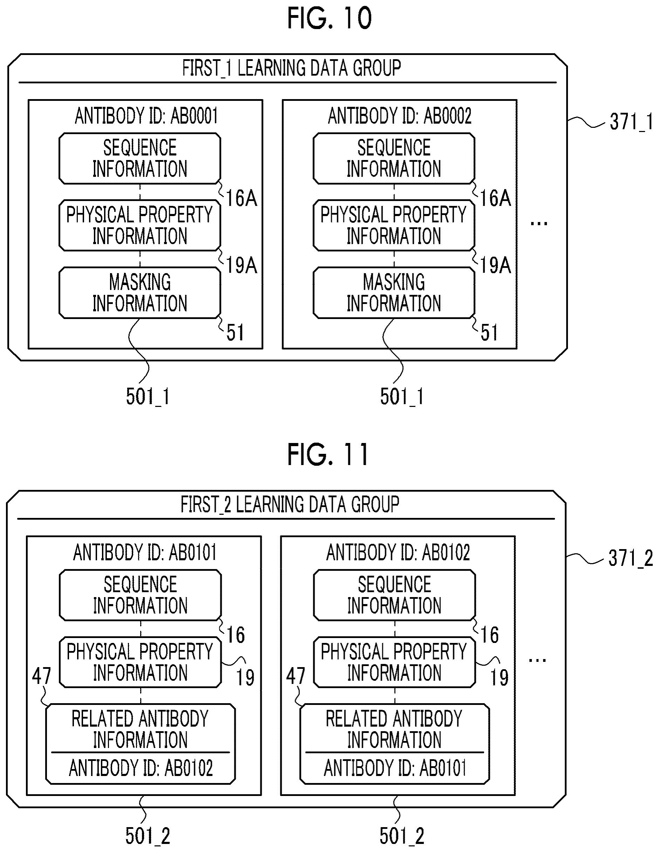

As shown in FIG. 10 as an example, first_1 learning data 501_1 constituting the first_1 learning data group 371_1 includes the sequence information 16A, the physical property information 19A, and masking information 51. The masking information 51 is information indicating which portion of the alphabet of the sequence information 16 and the standard free energy change ΔG, the solvent accessible surface area SASA, and the charge state of the physical property information 19 is masked by the masking process. That is, the masking information 51 is information that is an answer to the MLM of the pre-training. The sequence information 16A and the physical property information 19A of the first_1 learning data 501_1 are examples of "learning sequence information" and "learning physical property information" according to the disclosed technology.

As shown in FIG. 11 as an example, first_2 learning data 501_2 constituting the first_2 learning data group 371_2 includes the sequence information 16, the physical property information 19, and the related antibody information 47. The related antibody information 47 is information that is an answer to the NSP of the pre-training. The sequence information 16 and the physical property information 19 of the first_2 learning data 501_2 are also examples of "learning sequence information" and "learning physical property information" according to the disclosed technology.

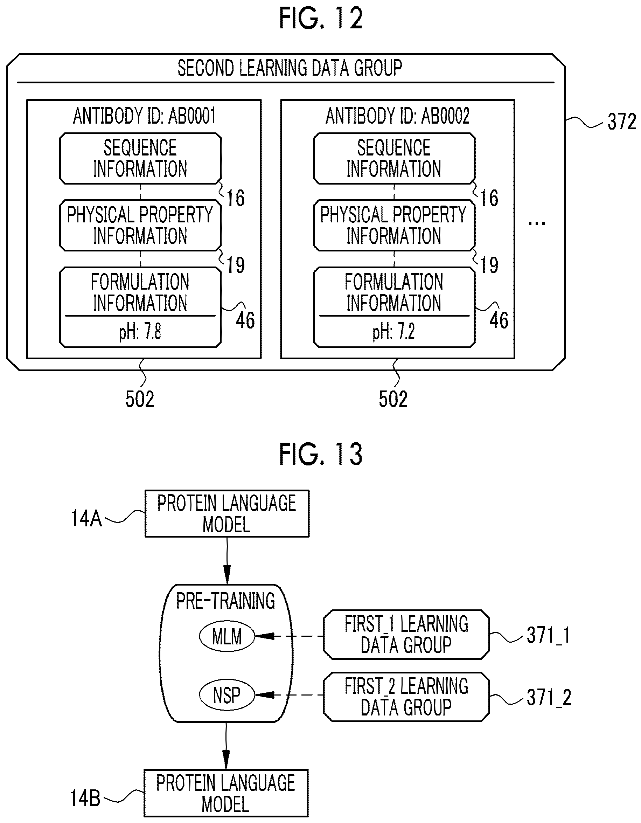

As shown in FIG. 12 as an example, second learning data 502 constituting the second learning data group 372 includes the sequence information 16, the physical property information 19, and the formulation information 46. The formulation information 46 is information that is an answer to the FT. The sequence information 16 and the physical property information 19 of the second learning data 502 are also examples of "learning sequence information" and "learning physical property information" according to the disclosed technology. In the following description, the first_1 learning data 501_1, the first_2 learning data 501_2, and the second learning data 502 are collectively referred to as learning data 50 in a case in which it is not necessary to particularly distinguish between the first_1 learning data 501_1, the first_2 learning data 501_2, and the second learning data 502.

As shown in FIG. 13 as an example, the pre-training unit 42 performs the MLM by using the first_1 learning data group 371_1 as the pre-training. In addition, the pre-training unit 42 performs the NSP by using the first_2 learning data group 371_2 as the pre-training. By performing the pre-training in this way, the pre-training unit 42 sets the protein language model 14A to the protein language model 14B.

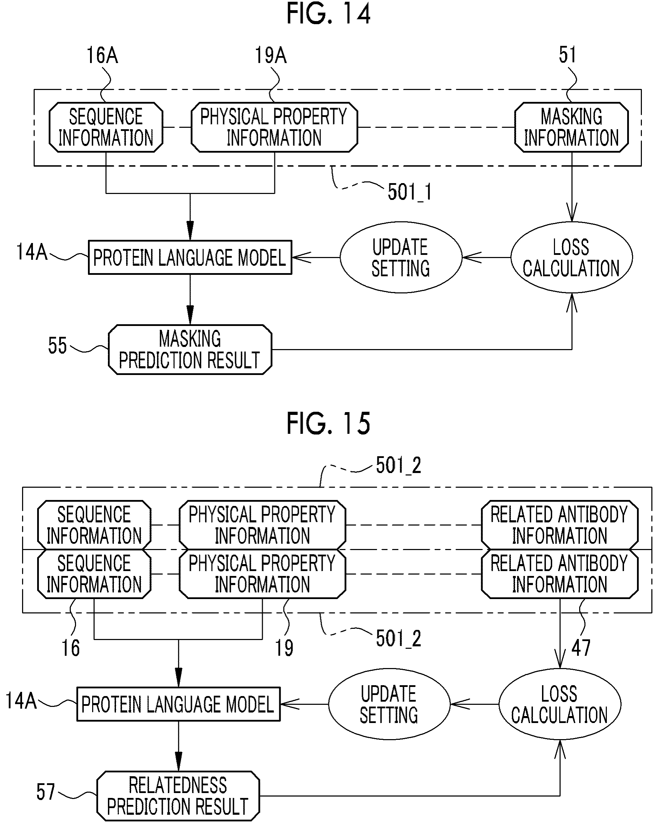

As shown in FIG. 14 as an example, in the MLM of the pre-training, the pre-training unit 42 inputs the sequence information 16A and the physical property information 19A of the first_1 learning data 501_1 to the protein language model 14A and causes the protein language model 14A to output a masking prediction result 55. The masking prediction result 55 is a result of predicting which portion of the alphabet of the sequence information 16 and the standard free energy change ΔG, the solvent accessible surface area SASA, and the charge state of the physical property information 19 is masked by the masking process. The protein language model 14A performs a loss calculation using a loss function based on the masking prediction result 55 and the masking information 51. Then, the update setting of the value of each parameter of the protein language model 14A is performed according to the result of the loss calculation, and the protein language model 14A is updated according to the update setting.

The pre-training unit 42 repeatedly performs the series of processing of inputting the sequence information 16A and the physical property information 19A to the protein language model 14A, outputting the masking prediction result 55 from the protein language model 14A, performing the loss calculation, performing the update setting, and updating the protein language model 14A while changing the first_1 learning data 501_1 until the prediction accuracy of the masking prediction result 55 reaches a preset level. Alternatively, in a case in which the series of processing is repeated a preset number of times, the pre-training unit 42 ends the series of processing.

As shown in FIG. 15 as an example, in the NSP of the pre-training, the pre-training unit 42 inputs the sequence information 16 and the physical property information 19 of the two pieces of first_2 learning data 501_2 to the protein language model 14A and causes the protein language model 14A to output a relatedness prediction result 57. The relatedness prediction result 57 is a result of predicting whether or not there is relatedness to the antibody 15 related to the two pieces of first_2 learning data 501_2. The protein language model 14A performs a loss calculation using a loss function based on the relatedness prediction result 57 and the related antibody information 47. Then, the update setting of the value of each parameter of the protein language model 14A is performed according to the result of the loss calculation, and the protein language model 14A is updated according to the update setting.

The pre-training unit 42 repeatedly performs the series of processing of inputting the sequence information 16 and the physical property information 19 to the protein language model 14A, outputting the relatedness prediction result 57 from the protein language model 14A, performing the loss calculation, performing the update setting, and updating the protein language model 14A while changing the first_2 learning data 501_2 until the prediction accuracy of the relatedness prediction result 57 reaches a preset level. Alternatively, in a case in which the series of processing is repeated a preset number of times, the pre-training unit 42 ends the series of processing. As the pre-training, only the MLM shown in FIG. 14 may be performed, and the NSP shown in FIG. 15 may not be performed.

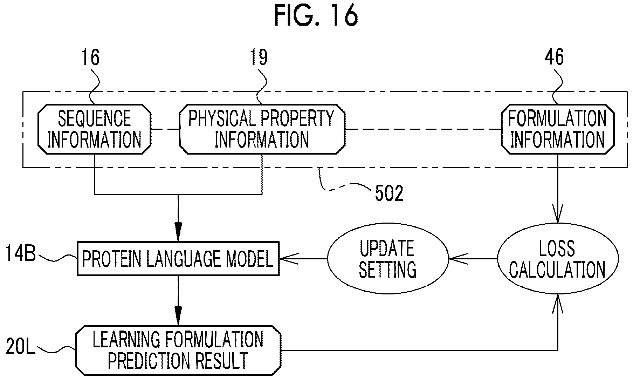

As shown in FIG. 16 as an example, the FT unit 43 inputs the sequence information 16 and the physical property information 19 of the second learning data 502 to the protein language model 14B and causes the protein language model 14B to output a learning formulation prediction result 20L. The protein language model 14B performs a loss calculation using a loss function based on the learning formulation prediction result 20L and the formulation information 46. Then, the update setting of the value of each parameter of the protein language model 14B is performed according to the result of the loss calculation, and the protein language model 14B is updated according to the update setting.

The FT unit 43 repeatedly performs the series of processing of inputting the sequence information 16 and the physical property information 19 to the protein language model 14B, outputting the learning formulation prediction result 20L from the protein language model 14B, performing the loss calculation, performing the update setting, and updating the protein language model 14B while changing the second learning data 502 until the prediction accuracy of the learning formulation prediction result 20L reaches a preset level. Alternatively, in a case in which the series of processing is repeated a preset number of times, the FT unit 43 ends the series of processing. In this way, the protein language model 14B in which the prediction accuracy of the learning formulation prediction result 20L has reached the preset level or the protein language model 14B in which the series of processing has been repeated a preset number of times is transmitted from the learning device 11 to the medicament development support device 12 as the protein language model 14C after the FT.

The sequence information 16 and the physical property information 19 (the sequence information 16A and the physical property information 19A in the case of FIG. 14) are converted into vector data in the protein language model 14. The vector data is data in which the alphabet of the sequence information 16, the standard free energy change ΔG, the solvent accessible surface area SASA, and the charge state of the physical property information 19 are represented by a multi-dimensional, for example, 64-dimensional vector, respectively.

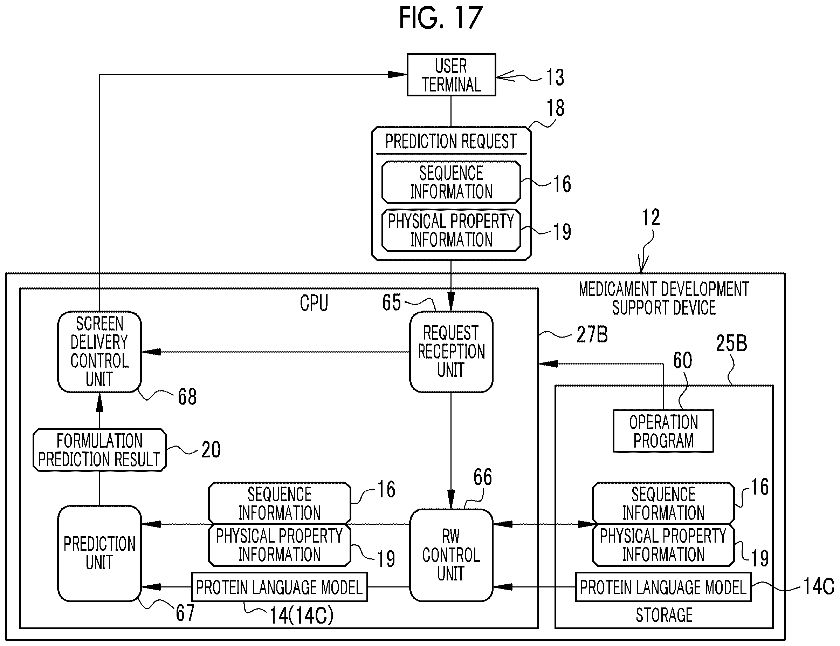

As shown in FIG. 17 as an example, an operation program 60 is stored in a storage 25B of the medicament development support device 12. The operation program 60 is an AP for causing the computer to function as the medicament development support device 12. That is, the operation program 60 is an example of an "operation program of a medicament development support device" according to the disclosed technology. The storage 25B also stores the protein language model 14C, the sequence information 16, the physical property information 19, and the like.

In a case in which the operation program 60 is activated, the CPU 27B of the computer constituting the medicament development support device 12 functions as a request reception unit 65, an RW control unit 66, a prediction unit 67, and a screen delivery control unit 68 in cooperation with the memory 26 and the like.

The request reception unit 65 receives various requests from the user terminal 13. In particular, the request reception unit 65 receives the prediction request 18 from the user terminal 13. As described above, the prediction request 18 includes the sequence information 16 and the physical property information 19. Therefore, the request reception unit 65 acquires the sequence information 16 and the physical property information 19 by receiving the prediction request 18. In a case in which the prediction request 18 is received, the request reception unit 65 outputs the sequence information 16 and the physical property information 19 included in the prediction request 18 to the RW control unit 66. In addition, the request reception unit 65 outputs the terminal ID of the user terminal 13 included in the prediction request 18 to the screen delivery control unit 68.

The RW control unit 66 controls the storage of various types of data in the storage 25B and the read-out of various types of data from the storage 25B. For example, the RW control unit 66 stores the protein language model 14C transmitted from the learning device 11 in the storage 25B. In addition, the RW control unit 66 stores the sequence information 16 and the physical property information 19 from the request reception unit 65 in the storage 25B. The RW control unit 66 reads out the sequence information 16 and the physical property information 19 from the storage 25B and outputs the read sequence information 16 and the physical property information 19 to the prediction unit 67. Further, the RW control unit 66 reads out the protein language model 14C from the storage 25B and outputs the read protein language model 14C to the prediction unit 67.

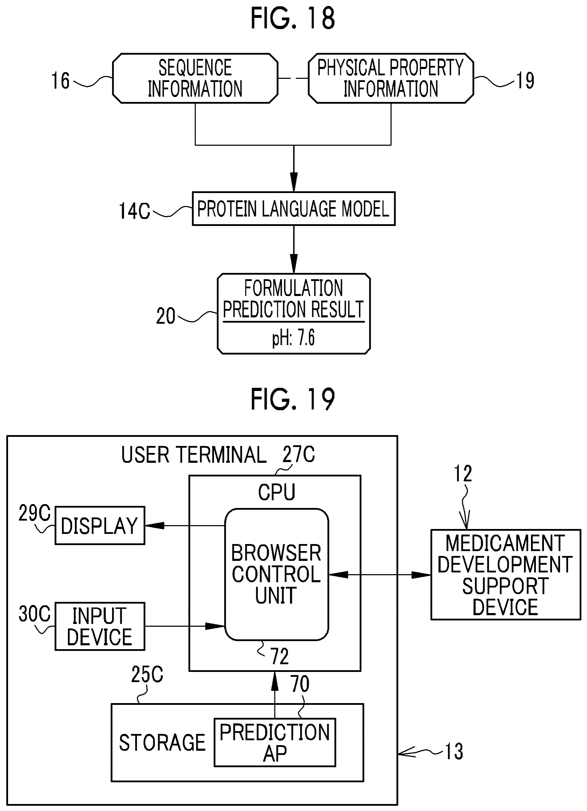

As shown in FIG. 18 as an example, the prediction unit 67 inputs the sequence information 16 and the physical property information 19 to the protein language model 14C and causes the protein language model 14C to output a formulation prediction result 20. The prediction unit 67 outputs the formulation prediction result 20 to the screen delivery control unit 68. The formulation prediction result 20 is a hydrogen ion exponent of the preservation solution here.

The screen delivery control unit 68 performs control of delivering various screens to the user terminal 13. Specifically, the screen delivery control unit 68 delivers output of the various screens to the user terminal 13 that is a transmitter of the various requests, in the form of screen data for web delivery created using a markup language such as extensible markup language (XML). In this case, the screen delivery control unit 68 specifies the user terminal 13 that is the transmission source of various requests based on the terminal ID from the request reception unit 65. Note that, instead of XML, another data description language, such as JavaScript (registered trademark) Object Notation (JSON), may be used.

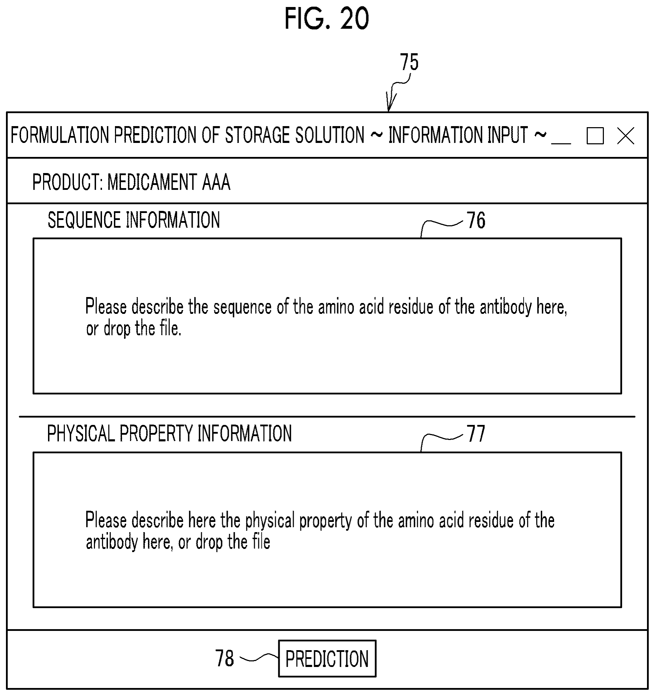

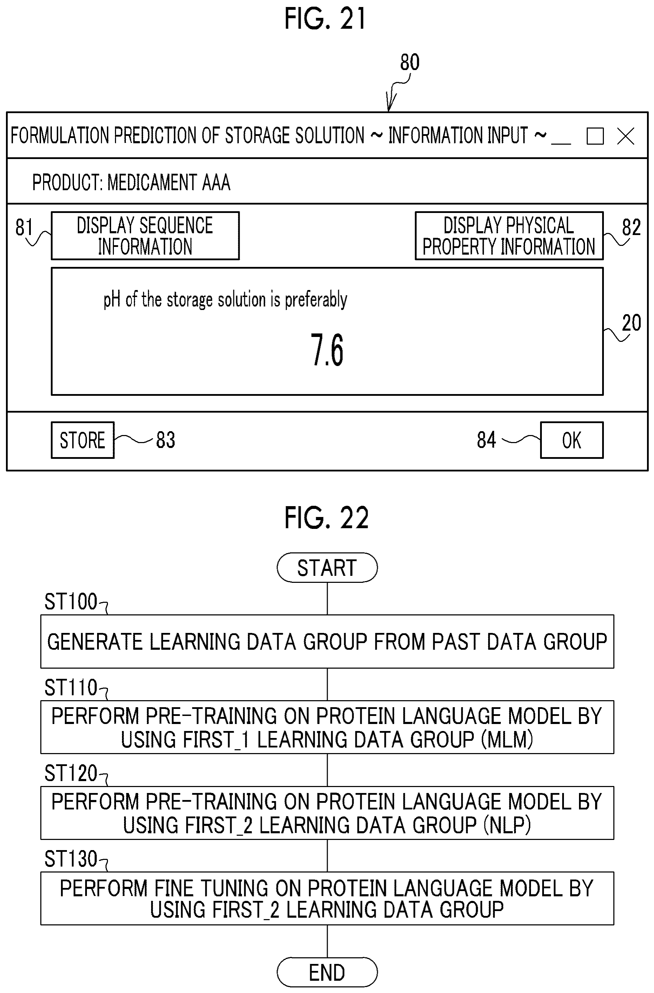

Various screens include an information input screen 75 (see FIG. 20) for inputting the sequence information 16 and the physical property information 19, a prediction result display screen 80 (see FIG. 21) for displaying the formulation prediction result 20, and the like. In addition, the CPU 27B is also constructed with an instruction reception unit that receives various operation instructions from the input device 30, in addition to each of the processing units 65 to 68.

As shown in FIG. 19 as an example, a prediction AP 70 is stored in a storage 25C of the user terminal 13. The prediction AP 70 is installed in the user terminal 13 by the user U. The prediction AP 70 is an AP for predicting information related to an appropriate formulation formula of a preservation solution of a biopharmaceutical. In a case in which the prediction AP 70 is activated, a CPU 27C of the user terminal 13 functions as a browser control unit 72 in cooperation with the memory 26 and the like. The browser control unit 72 controls an operation of a dedicated web browser of the prediction AP 70.

The browser control unit 72 reproduces various screens based on various screen data from the medicament development support device 12 and displays the reproduced various screens on the display 29C. Additionally, the browser control unit 72 receives various operation instructions input by the user U from the input device 30C through various screens. The browser control unit 72 transmits various requests in response to the operation instruction, including the prediction request 18, to the medicament development support device 12.

In a case in which the prediction AP 70 is activated, the information input screen 75 shown in FIG. 20 as an example is displayed on the display 29C under the control of the browser control unit 72. The information input screen 75 is provided with an input box 76 for the sequence information 16 and an input box 77 for the physical property information 19. In the input box 76, the sequence information 16 can be described or a file of the sequence information 16 can be dropped. Similarly, in the input box 77, the physical property information 19 can be described or a file of the physical property information 19 can be dropped.

The user U inputs desired sequence information 16 and physical property information 19 into the input boxes 76 and 77, and then selects a prediction button 78. In a case in which the prediction button 78 is selected, the browser control unit 72 generates the prediction request 18 including the sequence information 16 and the physical property information 19 input to the input boxes 76 and 77, and transmits the generated prediction request 18 to the medicament development support device 12.

In addition, in a case in which the prediction of the information related to the appropriate formulation formula of the preservation solution of the biopharmaceutical is performed in the medicament development support device 12, the prediction result display screen 80 shown in FIG. 21 as an example is displayed on the display 29C under the control of the browser control unit 72. On the prediction result display screen 80, the formulation prediction result 20, that is, a message representing the formulation prediction result 20 is displayed. As described above, the formulation prediction result 20 is presented to the user U in a form of delivery of screen data.

An sequence information display button 81 and a physical property information display button 82 are provided at an upper part of the prediction result display screen 80. In a case in which the sequence information display button 81 is selected, a display screen of the sequence information 16 is displayed in a pop-up manner. Similarly, in a case in which the physical property information display button 82 is selected, a display screen of the physical property information 19 is displayed in a pop-up manner.

In addition, a save button 83 and an OK button 84 are provided at a lower part of the prediction result display screen 80. In a case in which the save button 83 is selected, the sequence information 16, the physical property information 19, and the formulation prediction result 20 are stored in the storage 25C of the user terminal 13 in association with each other. In a case in which the OK button 84 is selected, the display of the prediction result display screen 80 is erased.

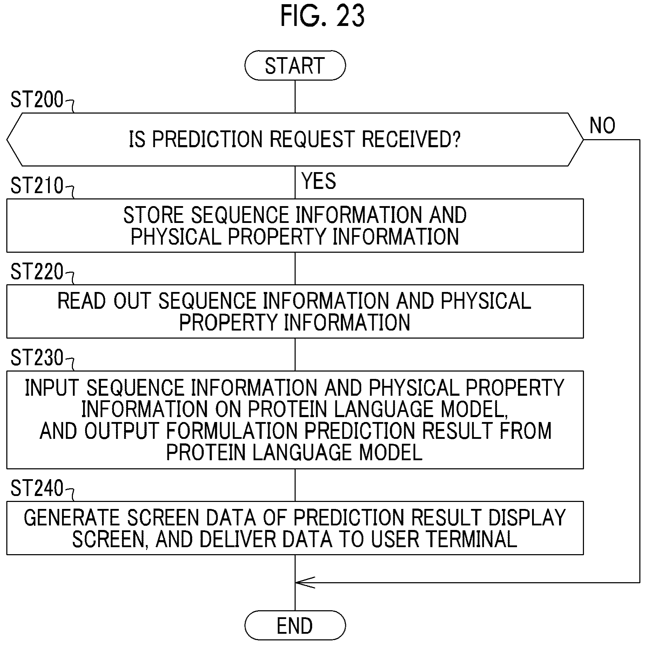

Next, the operation and effects of the above-described configuration will be described with reference to the flowcharts shown in FIGS. 22 and 23 as an example. In a case in which the operation program 35 is activated in the learning device 11, as shown in FIG. 5, the CPU 27A of the learning device 11 functions as the RW control unit 40, the generation unit 41, the pre-training unit 42, and the FT unit 43. In addition, in a case in which the operation program 60 is activated in the medicament development support device 12, as shown in FIG. 17, the CPU 27B of the medicament development support device 12 functions as the request reception unit 65, the RW control unit 66, the prediction unit 67, and the screen delivery control unit 68. Further, in a case in which the prediction AP 70 is activated in the user terminal 13, as shown in FIG. 19, the CPU 27C of the user terminal 13 functions as the browser control unit 72.

As shown in FIG. 22, in the learning device 11, as shown in FIGS. 7 to 12, the generation unit 41 generates the learning data group 37 from the past data group 36 (step ST100). The learning data 50 constituting the learning data group 37 includes the sequence information 16 and the physical property information 19. Therefore, by generating the learning data group 37 by the generation unit 41, the learning device 11 acquires the learning data 50 including the sequence information 16 and the physical property information 19. The learning data group 37 is output from the generation unit 41 to the RW control unit 40, and is stored in the storage 25A under the control of the RW control unit 40.

The RW control unit 40 reads out the protein language model 14A and the first learning data group 371 from the storage 25A, and outputs the read protein language model 14A and the first learning data group 371 to the pre-training unit 42. As shown in FIGS. 13 to 15, in the pre-training unit 42, the MLM is performed on the protein language model 14A as the pre-training by using the first_1 learning data group 371_1 (step ST110). In addition, the NSP is performed on the protein language model 14A as the pre-training by using the first_2 learning data group 371_2 (step ST120). The protein language model 14B after the pre-training is output from the pre-training unit 42 to the RW control unit 40, and is stored in the storage 25A under the control of the RW control unit 40.

The RW control unit 40 reads out the protein language model 14B and the second learning data group 372 from the storage 25A, and outputs the read protein language model 14B and the second learning data group 372 to the FT unit 43. As shown in FIG. 16, in the FT unit 43, the FT is performed on the protein language model 14B by using the second learning data group 372 (step ST130). The protein language model 14C after the FT is output from the FT unit 43 to the RW control unit 40, and is stored in the storage 25A under the control of the RW control unit 40. Then, the protein language model 14C is transmitted from the learning device 11 to the medicament development support device 12, and is stored in the storage 25B of the medicament development support device 12.

The information input screen 75 shown in FIG. 20 is displayed on the display 29C of the user terminal 13 under the control of the browser control unit 72. In a case in which the user U inputs the desired sequence information 16 and the physical property information 19 into the input boxes 76 and 77 and selects the prediction button 78 on the information input screen 75, the prediction request 18 is transmitted from the browser control unit 72 to the medicament development support device 12. As shown in FIG. 1, the prediction request 18 includes the sequence information 16 and the physical property information 19, and the terminal ID of the user terminal 13.

As shown in FIG. 23, in the medicament development support device 12, the prediction request 18 is received by the request reception unit 65, so that the sequence information 16 and the physical property information 19 included in the prediction request 18 are acquired (YES in step ST200). The sequence information 16 and the physical property information 19 included in the prediction request 18 are output from the request reception unit 65 to the RW control unit 66, and are stored in the storage 25B under the control of the RW control unit 66 (step ST210). In addition, the terminal ID of the user terminal 13 included in the prediction request 18 is output from the request reception unit 65 to the screen delivery control unit 68.

The sequence information 16 and the physical property information 19 are read out from the storage 25B by the RW control unit 66 (step ST220). The sequence information 16 and the physical property information 19 are output from the RW control unit 66 to the prediction unit 67. In addition, the protein language model 14C is read out from the storage 25B by the RW control unit 66, and the read protein language model 14C is output to the prediction unit 67.

As shown in FIG. 18, in the prediction unit 67, the sequence information 16 and the physical property information 19 are input to the protein language model 14C. As a result, the formulation prediction result 20 is output from the protein language model 14C (step ST230). The formulation prediction result 20 is output to the screen delivery control unit 68 from the prediction unit 67.

The screen delivery control unit 68 generates screen data of the prediction result display screen 80 shown in FIG. 21 based on the formulation prediction result 20. The screen data of the prediction result display screen 80 is delivered to the user terminal 13 that is the transmission source of the prediction request 18 under the control of the screen delivery control unit 68 (step ST240).

In the user terminal 13, the screen data of the prediction result display screen 80 is reproduced under the control of the browser control unit 72, and the reproduced prediction result display screen 80 is displayed on the display 29C. As a result, the formulation prediction result 20 is presented to the user U.

As described above, the CPU 27A of the learning device 11 comprises the generation unit 41, the pre-training unit 42, and the FT unit 43. The generation unit 41 acquires the learning data 50 including the sequence information 16 of the amino acid residues constituting the antibody 15 included in the biopharmaceutical and the physical property information 19 of the amino acid residues. The pre-training unit 42 and the FT unit 43 perform the pre-training and the fine-tuning of the protein language model 14 that outputs the formulation prediction result 20 by using the learning data 50.

In addition, the CPU 27B of the medicament development support device 12 comprises the request reception unit 65, the prediction unit 67, and the screen delivery control unit 68. The request reception unit 65 acquires the sequence information 16 and the physical property information 19 by receiving the prediction request 18. The prediction unit 67 uses the protein language model 14C that has been subjected to the pre-training and the fine-tuning by using the learning data 50 including the learning sequence information 16 and the learning physical property information 19. The prediction unit 67 inputs the sequence information 16 and the physical property information 19 to the protein language model 14C and causes the protein language model 14C to output the formulation prediction result 20. The screen delivery control unit 68 presents the formulation prediction result 20 to the user U by delivering the screen data of the prediction result display screen 80 including the formulation prediction result 20 to the user terminal 13. Since the physical property information 19 is also considered in addition to the sequence information 16, it is possible to improve the prediction accuracy of the protein language model 14C as compared with a case in which only the sequence information 16 is input to the protein language model 14C.

As shown in FIGS. 8, 9, 13, and 14, the protein language model 14C is a model in which the MLM is performed as the pre-training by using the first_1 learning data 501_1 in which a portion of the sequence information 16 and a portion of the physical property information 19 are masked to be the sequence information 16A and the physical property information 19A. Therefore, it is possible to generate the protein language model 14C that can refer to not only the sequence information 16 but also the physical property information 19.

The physical property information 19 is the standard free energy change ΔG as a numerical value representing hydrophilicity/hydrophobicity of the amino acid residue, the solvent accessible surface area SASA of the amino acid residue, and data representing a charge state of the amino acid residue. All of these greatly affect an appropriate formulation formula or the like for stabilizing the quality of the biopharmaceutical. Therefore, the prediction accuracy of the protein language model 14C can be significantly improved. The physical property information 19 may include at least one of a numerical value representing hydrophilicity/hydrophobicity of the amino acid residue, the solvent accessible surface area SASA of the amino acid residue, or data representing a charge state of the amino acid residue.

The prediction result is the formulation prediction result 20 including information related to the formulation formula of the preservation solution of the biopharmaceutical. Therefore, it is possible to contribute to the stable maintenance of the quality of the biopharmaceutical. The information related to the formulation formula may be a temperature of the preservation solution, a type of an additive added to the preservation solution, a concentration of the additive, or the like, instead of or in addition to the example of the hydrogen ion exponent. In addition, the prediction result is not limited to the information related to the formulation formula. For example, the degree of aggregation of the antibody 15 may be used.

The medicament including the antibody 15 as the protein is called an antibody drug and is widely used not only for the treatment of chronic diseases, such as cancer, diabetes, and rheumatoid arthritis, but also for the treatment of rare diseases such as hemophilia and a Crohn's disease. Therefore, according to the present example in which the protein is the antibody 15, it is possible to further promote the development of antibody drugs widely used for the treatment of various diseases.

The protein is not limited to the example of the antibody 15. Examples of the cell product include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (seventh factor, eighth factor, ninth factor, or the like), an enzyme (lysosomal enzyme, deoxyribonucleic acid (DNA) degrading enzyme, or the like), a fragment crystallizable (Fc) fusion protein, a receptor, albumin, and a protein vaccine. In addition, examples of the antibody include a bispecific antibody, an antibody-drug conjugate, a low-molecular-weight antibody, a sugar-chain-modified antibody, and the like.

In addition, the substance derived from amino acids is not limited to the protein. The substance derived from amino acids may be a peptide, a nucleic acid, or the like. Therefore, the medicament is not limited to a biopharmaceutical that requires a biotechnology such as a gene recombination technology and a cell culture technology, and may be a peptide medicament, a nucleic acid medicament, or the like that does not require a biotechnology and can be manufactured by only a chemical synthesis technology.

As the physical property information 19, identification data of the amino acid residues as an acid or a base, a dissociation constant, or the like may be adopted. In addition, as the data representing the charge state of the amino acid residues, a spatial charge map (SCM) or the like may be adopted.

In addition to the sequence information 16 and the physical property information 19, information on a secondary structure, a tertiary structure, and a quaternary structure of the protein may be input to the protein language model 14.

The protein language model 14C may continue to be trained even after being stored in the storage 25B of the medicament development support device 12.

Although an example has been described in which the generation unit 41 of the learning device 11 generates the learning data group 37, the present disclosure is not limited thereto. The learning data group 37 may be generated by a device different from the learning device 11, and the learning data group 37 may be transmitted to the learning device 11 from the different device. In addition, a part or all of the functions of the learning device 11 may be performed by the medicament development support device 12. Similarly, a part or all of the functions of the medicament development support device 12 may be performed by the user terminal 13.

The learning device 11 and the medicament development support device 12 may be installed in the pharmaceutical company or the CRO, or may be installed in a data center independent of the pharmaceutical company or the CRO.

The formulation prediction result 20 itself may be delivered to the user terminal 13 instead of delivering the screen data of the prediction result display screen 80 including the formulation prediction result 20 to the user terminal 13. In this case, in the user terminal 13, the prediction result display screen 80 is generated based on the formulation prediction result 20 under the control of the browser control unit 72.

The method of presenting the formulation prediction result 20 to the user U is not limited to the presentation by the delivery of the example of the screen data. The formulation prediction result 20 may be presented to the user U by printing the formulation prediction result 20 on a paper medium, or may be presented to the user terminal 13 by attaching the formulation prediction result 20 to an electronic mail.

The hardware configuration of the computer constituting the learning device 11 and the medicament development support device 12 according to the disclosed technology can be variously modified. For example, the learning device 11 and the medicament development support device 12 can be configured by a plurality of computers separated as hardware in order to improve processing capability and reliability. For example, the functions of the RW control unit 40 and the generation unit 41 and the functions of the pre-training unit 42 and the FT unit 43 are distributed to two computers. In this case, the learning device 11 is configured by two computers. Alternatively, the functions of the request reception unit 65 and the RW control unit 66 and the functions of the prediction unit 67 and the screen delivery control unit 68 are distributed to two computers. In this case, the medicament development support device 12 is configured by two computers.

As described above, the hardware configuration of the computer of the learning device 11 and the medicament development support device 12 can be appropriately changed according to the required performance such as processing capability, safety, and reliability. Not only the hardware but also the APs such as the operation program 35 and 60 may be duplicated or stored in a distributed manner between a plurality of storages for the purpose of securing safety and reliability.

In the above-described embodiment, for example, as a hardware structure of a processing unit that executes various types of processing, such as the RW control units 40 and 66, the generation unit 41, the pre-training unit 42, the FT unit 43, the request reception unit 65, the prediction unit 67, the screen delivery control unit 68, and the browser control unit 72, various processors shown below can be used. The various processors include, in addition to the CPUs 27A, 27B, 27C that are general-purpose processors functioning as various processing units by executing software (the operation program 35 and 60, and the prediction AP 70) as described above, a programmable logic device (PLD) such as a field programmable gate array (FPGA) that is a processor having a circuit configuration changeable after manufacture, a dedicated electric circuit such as an application specific integrated circuit (ASIC) that is a processor having a circuit configuration dedicatedly designed to execute specific processing, and the like.

One processing unit may be configured by one of the various processors or by a combination of two or more processors of the same type or different types (for example, a combination of a plurality of FPGAs and/or a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. Second, as represented by a system on a chip (SoC) or the like, there is a form in which a processor, which implements the functions of the entire system including the plurality of processing units with a single integrated circuit (IC) chip, is used. As described above, the various processing units are configured by using one or more of the above various processors as the hardware structure.

In addition, more specifically, an electric circuit (circuitry) in which circuit elements, such as semiconductor elements, are combined can be used as the hardware structure of these various processors.

The technology according to the following appendices can be perceived from the above description.

### [Supplementary Note 1]

A medicament development support device comprising:
a processor,
wherein the processor
   acquires sequence information of amino acid residues constituting a substance derived from amino acids included in a medicament, and physical property information of the amino acid residues,
   uses a substance language model that has been subjected to pre-training and fine-tuning by using learning data including learning sequence information and learning physical property information, and
   inputs the sequence information and the physical property information to the substance language model to cause the substance language model to output a prediction result related to the substance, and
   presents the prediction result to a user.

### [Supplementary Note 2]

The medicament development support device according to Supplementary Note 1, wherein the substance language model is a model that has been subjected to the pre-training by using the learning data in which at least one of a portion of the sequence information or a portion of the physical property information is masked.

### [Supplementary Note 3]

The medicament development support device according to Supplementary Note 1 or 2, wherein the physical property information is at least one of a numerical value representing hydrophilicity/hydrophobicity of the amino acid residue, a surface exposure area of the amino acid residue, or data representing a charge state of the amino acid residue.

### [Supplementary Note 4]

The medicament development support device according to any one of Supplementary Notes 1 to 3, wherein the prediction result includes information related to a formulation of a preservation solution of the medicament.

### [Supplementary Note 5]

The medicament development support device according to any one of Supplementary Notes 1 to 4, wherein the substance is any of a protein, a peptide, or a nucleic acid.

### [Supplementary Note 6]

The medicament development support device according to Supplementary Note 5, wherein the protein is an antibody.

### [Supplementary Note 7]

A learning device comprising:
a processor,
wherein the processor
   acquires learning data including sequence information of amino acid residues constituting a substance derived from amino acids included in a medicament, and physical property information of the amino acid residues, and
   performs pre-training and fine-tuning of a substance language model that outputs a prediction result related to the substance by using the learning data.

### [Supplementary Note 8]

The learning device according to Supplementary Note 7, wherein the learning data used for the pre-training is data in which at least one of a portion of the sequence information or a portion of the physical property information is masked.

The technology of the present disclosure can also be combined with various embodiments and/or various modification examples described above, as appropriate. The disclosed technology is not limited to the above embodiment and may adopt various configurations without departing from its gist. Furthermore, the technology of the present disclosure extends to a storage medium that non-transitorily stores the program, and a computer program product including the program, in addition to the program.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In addition, in order to avoid complications and facilitate grasping the parts according to the technology of the present disclosure, in the above-described contents and the above-shown contents, the description of technical general knowledge and the like that do not particularly require description for enabling the implementation of the technology of the present disclosure are omitted.

In the present specification, "A and/or B" has the same meaning as "at least one of A or B". That is, "A and/or B" means that it may be only A, only B, or a combination of A and B. In addition, in the present specification, also in a case where three or more matters are expressed in association by "and/or", the same concept as "A and/or B" is applied.

All of the documents, the patent applications, and the technical standards described in the present specification are incorporated herein by reference to the same extent as in a case where each of the documents, patent applications, and technical standards is specifically and individually described by being incorporated by reference.

## Claims

1. A medicament development support device comprising:
a processor,
wherein the processor
acquires sequence information of amino acid residues constituting a substance derived from amino acids included in a medicament, and physical property information of the amino acid residues,
uses a substance language model that has been subjected to pre-training and fine-tuning by using learning data including learning sequence information and learning physical property information, and
inputs the sequence information and the physical property information to the substance language model to cause the substance language model to output a prediction result related to the substance, and
presents the prediction result to a user.

2. The medicament development support device according to claim 1, wherein the substance language model is a model that has been subjected to the pre-training by using the learning data in which at least one of a portion of the sequence information or a portion of the physical property information is masked.

3. The medicament development support device according to claim 1, wherein the physical property information is at least one of a numerical value representing hydrophilicity/hydrophobicity of the amino acid residue, a surface exposure area of the amino acid residue, or data representing a charge state of the amino acid residue.

4. The medicament development support device according to claim 1, wherein the prediction result includes information related to a formulation of a preservation solution of the medicament.

5. The medicament development support device according to claim 1, wherein the substance is any of a protein, a peptide, or a nucleic acid.

6. The medicament development support device according to claim 5, wherein the protein is an antibody.

7. An operation method of a medicament development support device, the operation method comprising:
acquiring sequence information of amino acid residues constituting a substance derived from amino acids included in a medicament, and physical property information of the amino acid residues;
using a substance language model that has been subjected to pre-training and fine-tuning by using learning data including learning sequence information and learning physical property information; and
inputting the sequence information and the physical property information to the substance language model to cause the substance language model to output a prediction result related to the substance; and
presenting the prediction result to a user.

8. An operation program of a medicament development support device, the operation program causing a computer to execute a process comprising:
acquiring sequence information of amino acid residues constituting a substance derived from amino acids included in a medicament, and physical property information of the amino acid residues;
using a substance language model that has been subjected to pre-training and fine-tuning by using learning data including learning sequence information and learning physical property information; and
inputting the sequence information and the physical property information to the substance language model to cause the substance language model to output a prediction result related to the substance; and
presenting the prediction result to a user.

9. A learning device comprising:
a processor,
wherein the processor
acquires learning data including sequence information of amino acid residues constituting a substance derived from amino acids included in a medicament, and physical property information of the amino acid residues, and
performs pre-training and fine-tuning of a substance language model that outputs a prediction result related to the substance by using the learning data.

10. The learning device according to claim 9, wherein the learning data used for the pre-training is data in which at least one of a portion of the sequence information or a portion of the physical property information is masked.

11. An operation method of a learning device, the operation method comprising:
acquiring learning data including sequence information of amino acid residues constituting a substance derived from amino acids included in a medicament, and physical property information of the amino acid residues; and
performing pre-training and fine-tuning of a substance language model that outputs a prediction result related to the substance by using the learning data.

12. An operation program of a learning device, the operation program causing a computer to execute a process comprising:
acquiring learning data including sequence information of amino acid residues constituting a substance derived from amino acids included in a medicament, and physical property information of the amino acid residues; and
performing pre-training and fine-tuning of a substance language model that outputs a prediction result related to the substance by using the learning data.
